(19) Europäisches Patentamt
European Patent Office
Office européen des brevets

(11) Numéro de publication: **0 148 399**
**B1**

(12) # FASCICULE DE BREVET EUROPÉEN

(45) Date de publication du fascicule du brevet: **13.01.88**

(51) Int. Cl.⁴: **C 07 C 35/44**, C 07 C 29/14

(21) Numéro de dépôt: **84114512.1**

(22) Date de dépôt: **30.11.84**

(54) **Alcool polycyclique, procédé pour sa préparation, son utilisation en tant qu'ingrédient parfumant et composition le contenant.**

(30) Priorité: **22.12.83 CH 6840/83**

(43) Date de publication de la demande:
**17.07.85 Bulletin 85/29**

(45) Mention de la délivrance du brevet:
**13.01.88 Bulletin 88/2**

(84) Etats contractants désignés:
**CH DE FR GB LI NL**

(56) Documents cité:
**TETRAHEDRON LETTERS, vol. 25, no. 27, 3 juillet 1984, pages 2877-2878, Oxford, GB; R.L. SNOWDEN et al: "Stereoselective synthesis of (4RS, 7RS)-7-methyltetracyclo (6.2.1.02, 7.02, 10) undecan-4-ol. A trace constituent of lavender oil"**
**JOURNAL OF THE AMERICAN CHEMICAL SOCIETY, vol. 105, no. 25, 14 décembre 1983, pages 7389-7395 *7390,* 7391; K. MLINARIC-MAJERSKI et al.: "The bond between inverted carbon atoms. Synthesis and chemistry of 2,4-methano-2,4-didehydroadamantane; A highly reactive (3.1.1) propellane"**

(73) Titulaire: **FIRMENICH SA, 1, route des Jeunes, CH-1211 Genève 8 (CH)**

(72) Inventeur: **Naef, Regula, 30, chemin Vert, CH- 1227 Carouge (CH)**
Inventeur: **Snowden, Roger Leslie, Dr., 5, chemin des Palettes, CH- 1212 Grand- Lancy (CH)**
Inventeur: **Morris, Anthony Francis, Villa "Lindos" 12, route Cantonale, CH- 1261 Gingins (CH)**

(74) Mandataire: **Salvadori, Giuseppe, Dr., c/o Firmenich S.A. Case Postale 239, CH- 1211 Genève 8 (CH)**

EP 0 148 399 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention a trait au domaine de la parfumerie; elle concerne plus particulièrement l'utilisation d'un alcool polycyclique nouveau de formule

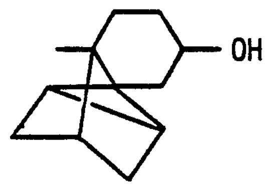

(I)

ou 7-méthyltétracyclo[6.2.1.$0^{2,7}$.$0^{2,10}$]undécan-4-ol.

Lors d'une analyse récente que nous avons effectuée sur l'huile essentielle de lavande (Lavendula officinalis, Chaix), nous avons constaté la présence d'un composé qui avait échappé aux nombreuses investigations effectuées jusqu'ici par divers groupes de recherche [voir à cet effet l'article de W. Hoffmann publié dans Seifen, Oele, Fette, Wachse 105, 287 (1979), ainsi que les références bibliographiques citées par cet auteur et R. Kaiser et al., Helv. Chim. Acta 66, 1835 et 1843, (1983)].

L'art antérieur fait état de la présence dans cette huile d'un nombre fort élevé de constituants, environ 140 selon certaines estimations. Notre analyse a permis de déterminer que ce nombre était encore supérieur. Nous avons en effet découvert pour la première fois qu'environ 160 autres composés y étaient présents, certains dans des proportions extrêmement petites, de l'ordre du ppm (partie par million). Parmi ces composés figure précisément le 7-méthyltétracyclo[6.2.1.$0^{2,7}$.$0^{2,10}$]undécan-4-ol, présent à une concentration inférieure à 1 ppm.

La nature de ce constituant aurait probablement également échappé à notre examen si nous n'avions pas observé, lors d'une étude indépendante conduite en parallèle, que l'alcool en question était présent en traces dans le 3-(2-exo-méthyl-3-méthylidène-bicyclo[2.2.1]hept-2-yl-propanal, produit intermédiaire pour la préparation d'épi-β-santalol [voir: Helv. Chim. Acta 64, 25 (1981)]. Nous disposions de ce fait de ses caractères analytiques et organoleptiques, ce qui nous permit, par déduction analogique, d'identifier le 7-méthyltétracyclo[6.2.1.$0^{2,7}$.$0^{2,10}$]undécan-4-ol dans l'huile essentielle de lavande à l'examen et d'attribuer sa structure correcte. La démarche qui préside à la présente invention nous a également amenés à formuler un procédé pour sa fabrication à partir précisément de 3-(2-exo-méthyl-3-méthylidène-bicyclo[2.2.1]hept-2-yl)-propanal, aldéhyde de formule

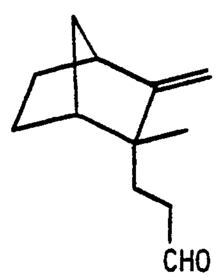

(II)

Ledit procédé, qui constitue également un des objets de la présente invention, consiste en la cyclisation catalytique de l'aldéhyde (II) en présence d'un réactif acide aprotique, en l'occurrence en présence d'un acide dit de Lewis. Parmi les acides de Lewis, il convient de mentionner tout particulièrement les halogénures d'aluminium, d'étain et de zinc, tels le chlorure d'étain, le chlorure ou le iodure de zinc, ou le trifluorure de bore. Ce dernier est utilisé de préférence. La cyclisation s'effectue en présence d'un solvant organique inerte dans les conditions de la réaction, et à cet effet, on utilise un hydrocarbure, de préférence halogéné. Selon un mode opératoire préférentiel, on utilise le dichlorométhane.

Le 3-(2-exo-méthyl-3-méthylidène-bicyclo[2.2.1]hept-2-yl)-propanal, produit de départ du procédé décrit ci-dessus, peut être obtenu à partir de norbornène suivant la méthode illustrée par le schéma réactionnel suivant:

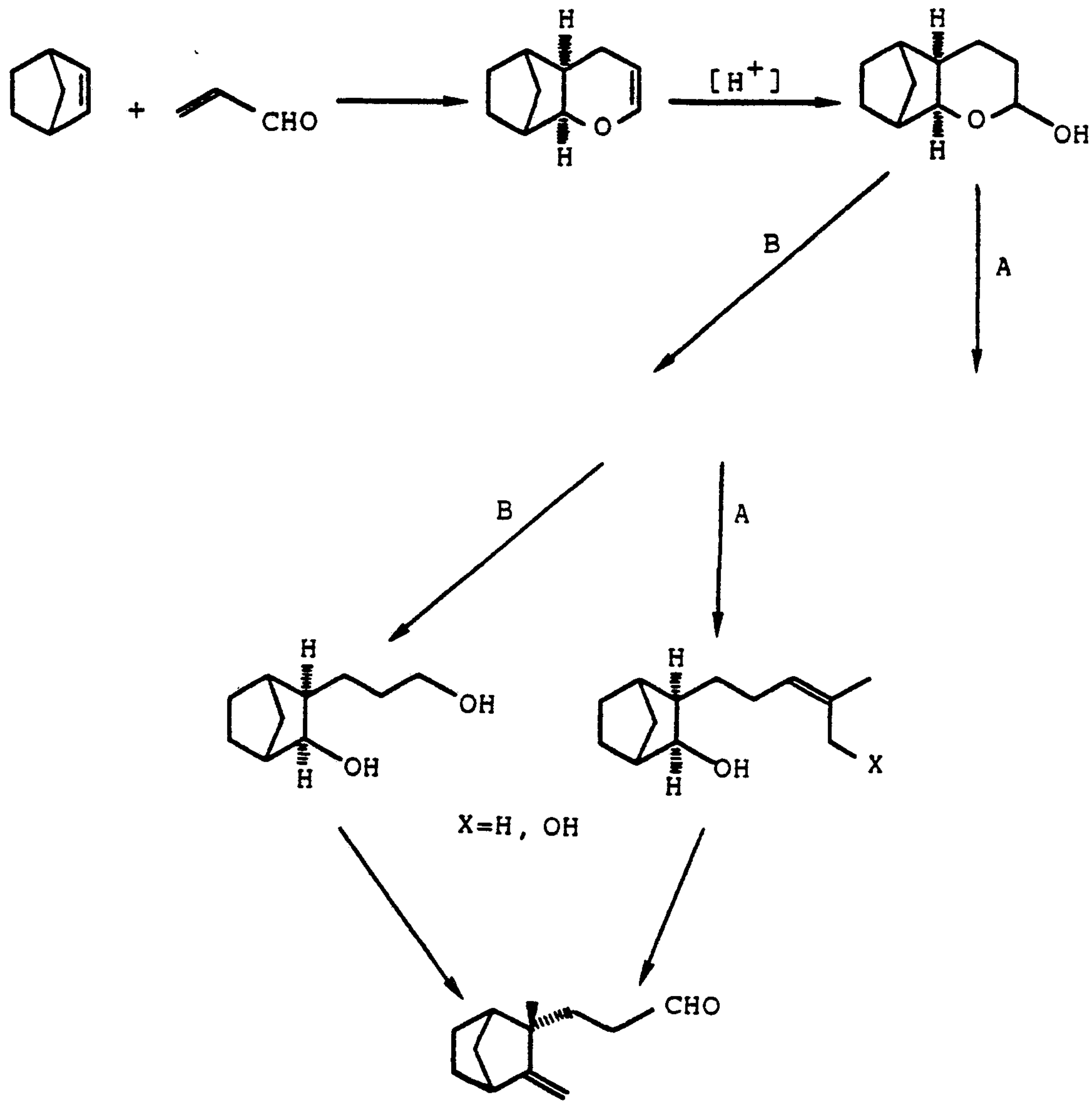


Le produit obtenu à l'aide du procédé décrit ci-dessus se présente sous une forme stéréoisomérique particulière, laquelle peut être définie par la formule suivante

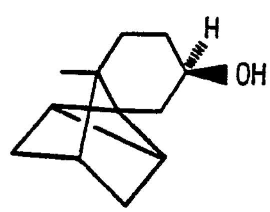

Il s'agit donc du (4RS,7RS)-7-méthyltétracyclo[6.2.1.$0^{2,7}$.$0^{2,10}$]undécan-4-ol. Or, c'est précisément sous cette forme isomérique que le produit se retrouve dans l'huile essentielle de lavande.

Les caractères olfactifs de ce produit rappellent ceux développés par l'essence de patchouli. Son aspect plus général boisé ressort à l'emploi dans des compositions variées et, de ce fait, il trouve une utilisation fort étendue dans la création de compositions destinées au parfumage d'articles aussi divers que les savons, les shampoings, les détergents, les adoucissants textiles, les crèmes de beauté, les désodorisants corporels ou d'air ambiant, ou les parfums alcooliques.

Les proportions dans lesquelles le produit de l'invention sert à développer les caractères odorants désirés peuvent varier dans une gamme de valeurs assez étendue. C'est ainsi que des concentrations aussi petites que 1 %, voire inférieures, peuvent être employées. Ces valeurs par ailleurs peuvent être augmentées jusqu'à 20 ou 30 % en poids par rapport au poids total de la composition dans laquelle il est incorporé. Lors du parfumage

d'articles tels les savons ou les détergents, par exemple, ces proportions sont inférieures aux valeurs indiquées et sont de l'ordre de 0,1-0,5 %.

Bien entendu, de telles proportions ne sauraient être interprétées de façon absolue et, comme souvent en pareil cas, l'homme de l'art sera amené, lors de la reproduction de l'invention, à rechercher les proportions les meilleures en fonction des effets odorants recherchés et en fonction de la nature des coingrédients présents dans une composition donnée ou de celle des articles que l'on désire parfumer, une telle démarche relevant de l'activité courante du parfumeur.

L'invention est illustrée de manière plus détaillée par les exemples qui suivent (températures en degrés centigrades).

**Exemple 1**

(4RS,7RS)-7-Méthyltétracyclo[6.2.1.$0^{2,7}$.$0^{2,10}$]undécan-4-ol

Une solution de 3-(2-exo-méthyl-3-méthylidène-bicyclo[2.2.1]hept-2-yl)-propanal (890 mg; 5mM) dans 5 ml de dichlorométhane a été ajoutée à une solution de $BF_3.Et_2O$ (50 mg) dans 10 ml de $CH_2Cl_2$ maintenue en agitation à -30° sous atmosphère d'azote. La température du mélange a augmenté ainsi graduellement en 1 h jusqu'à 0° et le mélage a été ensuite lavé avec une solution aqueuse saturée de $NaHCO_3$. La phase organique séparée a été séchée sur du $Na_2SO_4$ anhydre et le solvant a été évaporé, puis on a procédé à une purification du produit obtenu par chromatographie sur colonne remplie (50 g de $SiO_2$; éluant: $CH_2Cl_2$) suivie de sublimation. On a ainsi pu obtenir 555 mg (rend. 62 %) d'un solide cristallin ayant F 80-1°; $R_F$ ($CH_2Cl_2$): 0,21.

IR ($CDCl_3$): 3615, 3460, 1440, 1380, 1290, 1200, 1150, 1062, 1036, 1016, 860, et 810 $cm^{-1}$;

RMN: 0,87 (3H, s); 0,80-2,20 (13H); 2,10 (1H, s); 4,06 (1H, m) δ ppm;

SM: 178(48), 160(28), 145(60), 131(38), 119(52), 105(46), 91(100), 79(54), 67(35), 55(22), 41(50).

Le 3-(2-exo-méthyl-3-méthylidène-bicyclo[2.2.1]hept-2-yl)-propanal, utilisé comme produit de depart dans le procédé décrit ci-dessus, a été préparé conformement à la méthode décrite dans Helv. Chim. Acta 64, 25 (1981).

**Exemple 2**

Une composition parfumante de base de type lavande, fleuri, fougère, a été préparée en mélangeant les ingrédients suivants (parties en poids):

| | |
|---|---|
| Coumarine | 800 |
| Exaltex ® 1) 2) | 800 |
| Essence de géranium synth. | 1000 |
| Hedione ® 1) 3) | 600 |
| cis-Jasmone à 10 %* | 600 |
| Essence de lavande | 3000 |
| Lilial ® 4) | 500 |
| Lyral ® 5) | 200 |
| Mayol ® 6) 1) | 1000 |
| Mousse de chêne 50 %* | 100 |
| Composé obtenu suivant l'ex. 1 | 400 |
| | 9000 |

* dans le phtalate de diéthyle
1) origine: Firmenich SA, Genève
2) pentadécanolide
3) dihydrojasmonate de méthyle
4) origine: L. Givaudan; p-tert-butyl-α-méthyl-hydroxy-cinnamaldéhyde
5) origine: IFF; 4-(4-méthyl-4-hydroxyamyl)-3-cyclohexène carboxaldéhyde
6) hydroxy méthyl-p-isopropyl cyclohexane

L'adjonction du produit obtenu à l'exemple 1 confère à la composition de base un effet boisé particulièrement riche tout en rehaussant un caractère élégant et naturel de la note de lavande.

**Exemple 3**

Une composition parfumante de base de type rosé, fleuri, a été préparée en mélangeant les ingrédients suivants (parties en poids):

| | |
|---|---|
| Aldéhyde undécylénique à 50 %* | 300 |
| Acétate de benzyle | 800 |
| Salicylate de benzyle | 800 |
| Citronellol | 1000 |
| Dorinia® $_{SA}$ 1) | 200 |
| Exaltex ® 1) | 500 |
| Géraniol | 1500 |
| Iralia ® 1) | 500 |
| Linalol | 700 |
| Alcool phényléthylique | 1000 |
| Ylang | 500 |
| Composé obtenu suivant l'ex. 1 | 500 |
| | 8300 |

* dans le phtalate diéthylique
1) origine: Firmenich SA, Genève

L'adjonction du produit obtenu à l'exemple 1 confère à la composition de base un effet boisé particulièrement riche et sert à développer un caractère poudreux.

Les compositions ainsi obtenues aux exemples 2 et 3 ci-dessus se prêtent parfaitement au parfumage de shampoings et adoucissants textiles, le produit de l'invention étant parfaitement stable dans ces milieux.

**Exemple 4**

Une base adoucissante textile a été préparée en mélangeant les ingrédients suivants (parties en poids):

| Ingrédients | Partie | Origine |
|---|---|---|
| Praepagen WK | 10,0 | Hoechst |
| Emulsifiant O 120 | 0,5 | Zschimmer & Schwarz |
| Polyglycol 400 | 2,0 | Hoechst |
| Eau distillée | 84,4 | |
| Colorant: | | |
| Brilliant blau R 28032 | | |
| solution aqueuse 0,5 % | 0,1 | Siegle |
| Chlorure de sodium | | |
| solution aqueuse 10 % | 0,7 | |
| Poromycen F 10 | 0,1 | Kraft |
| Alcool isopropylique C+ | 2,0 | Shell |
| | 99,8 | |

Lorsqu'on ajoute à la base ainsi obtenue 0,2 parties en poids d'une des compositions préparées conformément aux exemples 2 et 3 ci-dessus, on obtient une base adoucissante parfumée au caractère odorant stable et plaisant.

**Revendications**

1. Composé de formule

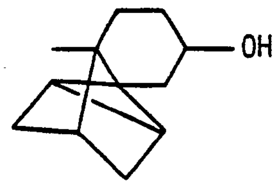

(I)

2. A titre de composé selon la revendication 1, le (4RS,7RS)-7-méthyltétracyclo[6.2.1.$0^{2,7}$.$0^{2,10}$]undécan-4-ol.

3. Procédé pour la préparation d'un composé selon la revendication 2, caractérisé en ce qu'on soumet à une cyclisation catalytique un aldéhyde de formule

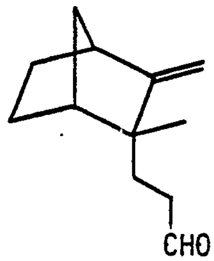

(II)

en présence d'un réactif acide aprotique.

4. Procédé selon la revendication 3, caractérisé en ce qu'on utilise comme réactif acide aprotique un halogénure de zinc ou le trifluorure de bore.

5. Procédé selon la revendication 4, caractérisé en ce que la cyclisation s'effectue en présence d'un solvant organique inerte dans les conditions de la réaction et à une température comprise entre -30 et 0° C.

6. Procédé selon la revendication 5, caractérisé en ce qu'on utilise comme solvant organique inerte le dichlorométhane.

7. Utilisation du composé selon la revendication 2 à titre d'ingrédient parfumant pour la préparation de parfums, de compositions parfumantes et de produits parfumés.

8. Composition parfumante résultant de l'utilisation selon la revendication 7.

**Patentansprüche**

1. Verbindung der Formel

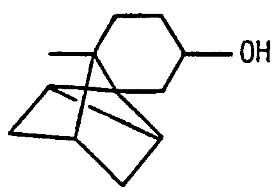

(I)

2. (4RS,7RS)-7-methyltetracyclo[6.2.1.$0^{2,7}$.$0^{2,10}$]undecan-4-ol als Verbindung gemäss Anspruch 1.

3. Verfahren zur Herstellung einer Verbindung gemäss Anspruch 2, dadurch gekennzeichnet, dass man einen Aldehyd der Formel

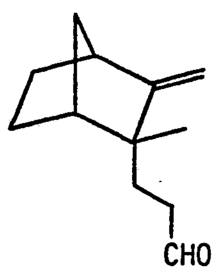

(II)

in Anwesenheit eines aprotischen Reagenzes katalytisch cyclisiert.

4. Verfahren gemäss Anspruch 3, dadurch gekennzeichnet, dass man ein Zinc-Halogenid oder Bortrifluorid als aprotisches Reagenz verwendet.

5. Verfahren gemäss Anspruch 4, dadurch gekennzeichnet, dass die Cyclisierung in Anwesenheit eines unter den Reaktionsbedingungen inerten organischen Lösungsmittels bei einer Temperatur von -30 bis 0°C durchgeführt wird.

6. Verfahren gemäss Anspruch 5, dadurch gekennzeichnet, dass man als inertes organisches Lösungsmittel Dichloromethan verwendet.

7. Verwendung der Verbindung gemäss Anspruch 2 als Parfümbestandteil zur Herstellung von Parfümen, Parfümkompositionen und parfümierten Produkten.
8. Parfümkomposition die von der Verwendung gemäss Anspruch 7 resultiert.

## Claims

1. Compound of formula

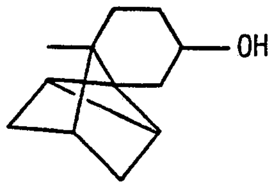

(I)

2. As compound according to claim 1, (4RS,7RS)-7-methyltetracyclo[$6.2.1.0^{2,7}.0^{2,10}$]undecan-4-ol.
3. Process for the preparation of a compound according to claim 2, characterized in that an aldehyde of formula

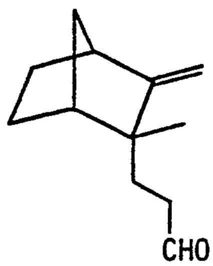

(II)

is catalytically cyclized in the presence of an aprotic acidic reagent.
4. Process according to claim 3, characterized in that there is used as aprotic acidic reagent a zinc halide or boron trifluoride.
5. Process according to claim 4, characterized in that the cyclization is effected in an organic solvent inert in the reaction conditions and at a temperature of between -30 and 0° C.
6. Process according to claim 5, characterized in that the inert organic solvent is dichloromethane.
7. Utilization of the compound according to claim 2 as perfuming ingredient for the preparation of perfumes, perfuming compositions and perfumed products.
8. Perfuming composition resulting from the utilization according to claim 7.